# EUROPÄISCHE PATENTANMELDUNG

(11) **EP 2 436 681 A1**
(43) Veröffentlichungstag der Anmeldung: **04.04.2012**
(21) Anmeldenummer: 10009238.6
(22) Anmeldetag: 06.09.2010
(51) Int. Cl.: C07D 317/22, C07D 319/06

(54) **Derivatisierung von Hydroxyacetalen und -acetalgemischen**

(71) Anmelder: Cognis IP Management GmbH, 40589 Düsseldorf (DE)
(72) Erfinder: Beller, Matthias, 18211 Nienhagen (DE); Kragl, Udo, 18198 Kritzmow (DE); Schörken, Ulrich, 40591 Düsseldorf (DE); Paetzold, Eckhard, 18184 Broderstorf (DE); Jackstell, Ralf, 27478 Cuxhafen (DE)
(74) Vertreter: Fabry, Bernd

(57) **Zusammenfassung**

Beschrieben wird ein Verfahren ausgehend von Hydroxyacetalen bzw. -gemischen zur katalytischen C-O-C-Knüpfungsreaktionen zur Etherbildung, speziell durch Telomerisation von Diensystemen, wobei Palladium-(0)-Komplexe als Katalysator genutzt, der Carbonylierung von Olefinen mit Rhodium-(0)-Komplexen und deren Reaktion mit den Hydroxyacetalen bzw. -gemischen, sowie die Dimerisierung der Hydroxyacetalen bzw. -gemischen mit Ruthenium-(0)-Komplexen unter Wasserabspaltung. Das Verfahren ermöglicht eine Verlängerung der Kohlenstoffkette des verwendeten Hydroxyacetals bzw. -gemisches um mehrere C-Atom und eine Erhöhung des Brennwertes der Produkte sowie eine Senkung der Verdampfungstemperatur. Die Produkte können als Brennstoffe, Lösungsmittel, Detergenzien, Kosmetika, Weichmacher, Stabilisatoren, Pharmaka, Fungizide und Mikrobiozide, Aromstoffe, Zwischenprodukte und als Additive für verschiedene Anwendungsgebiete eingesetzt werden.

## Beschreibung

Die Erfindung betrifft Verfahren zur katalytischen Umsetzung von Hydroxylgruppen haltigen Acetalen bzw. -gemischen mit Übergangsmetall-Phosphin-KomplexKatalysatoren, wobei die Hydroxylgruppe zu entsprechenden Ether- oder Ester-Gruppierungen der Acetalverbindungen umgesetzt werden.

In der DE 10 2008 009 103.0 wird gezeigt, dass durch eine bestimmte Reaktionsführung eine kostengünstige Stoffwandlung in einem Ein-Topf-Mehrphasensystem so zu gestalten ist, dass bei der Verwendung von Übergangsmetallkomplex-Katalysatoren eine Verlängerung um ein C-Atom der Olefine bei gleichzeitiger Erhöhung deren Reaktivität, Aldehyde synthetisiert sowie eine anschließende Reaktion mit Polyolen zu Acetalen ermöglicht wird. In einem Eintopfverfahren werden mittels einer vorgeschalteten Hydroformylierung mit Übergangsmetallkomplex-Katalysatoren werden Aldehyde synthetisiert, die in einer Folgereaktion mit Polyolen der zweiten wässrigen Phase wie z. B. Glycerin unter sauren Bedingungen acetalisiert werden.

Als nachteilig stellte sich aber für die Syntheseprodukte heraus, dass eine vollständige Umsetzung aller Hydroxylgruppen selbst mit kurzkettigen Aldehyden zu Acetalen mit Molmassen größer 300 führt. Bei Molmassen > 300 sind diese Produkte schwer zu händeln. Bei der Bildung von monocyclischen Acetalen bzw. -gemischen im Falle von Glycerin bleibt eine Hydroxylgruppe pro gebildetes Acetal übrig, die ungünstige Eigenschaften, wie zu hohe Siedepunkte und zu niedrige Heizwerte zur Folge haben.

In der unveröffentlichten Anmeldung EP 09 012 496.7 wird beschrieben wie alkoxylierte Glycerinacetale aus diesen hydroxylgruppenhaltigen Acetalen und gemischen durch Umsetzung mit Epoxiden synthetisiert werden können, die eine vielfältige Anwendbarkeit haben.

Das Ziel der war es nun, durch ein verbesserte Reaktionsführungen eine kostengünstige Stoffwandlung so zu gestalten, dass bei der Verwendung von Übergangsmetallkomplex-Katalysatoren eine Verlängerung um weitere C-Atom unter Etherbildung mit hydroxylgruppenhaltigen Acetalen und -gemischen ermöglicht wird.

Ein erster Gegenstand der vorliegenden Anmeldung betrifft daher ein Verfahren zur chemischen Reaktion von Hydroxyacetalen der allgemeinen Formel (I) 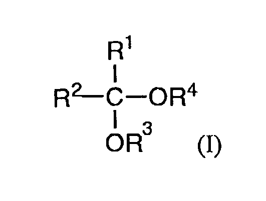 wobei die Gruppen R¹, R², R³ und R⁴ für lineare, verzweigte, cyclische und ggf. untereinander verknüpfte Alkylgruppen steht, mit der Maßgabe, dass mindestens eine der Gruppen R¹, R², R³ oder R⁴ eine freie Hydroxylgruppe aufweist, bzw. Gemische von Verbindungen der allgemeinen Formel (I) durch C-O-C- bzw. C-O-C=O-Knüpfungsreaktionen der Hydroxylfunktion der Verbindungen der allgemeinen Formel (I) oder deren Gemische in Gegenwart von Übergangsmetall-Phosphin- bzw. -Phosphit-Komplexkatalysatoren der 8. Nebengruppe in Ein- oder Mehrphasensystemen, wobei die Knüpfungsreaktion bei Temperaturen zwischen 0 und 200 °C durchgeführt wird, wobei für die Komplexkatalysatoren die Übergangsmetall/Phosphor-Verhältnisse zwischen 1 : 0,5 und 1 : 100, bevorzugt zwischen 1 : 1 bis 1 : 20 betragen.

Die hydroxylgruppenhaltigen Acetale bzw. -gemische werden C-O-C knüpfenden Reaktionen unterworfen. Als C-O-C knüpfenden Reaktionen werden Dimerisierungen unter Wasserabspaltung, Telomerisationen von Dienen mit nachfolgender Hydrierung, Carbonylierungen an Olefinen und Decarboxylierungen von Carbonsäuren und - derivaten angesehen, wobei es zur Bildung von Ethern kommt.

### Katalysatoren

Übergangsmetallkomplex-Katalysatoren werden für diese Reaktionen benötigt. Als Übergangsmetallkomplex-Katalysatoren werden als Zentralatom die Elemente der achten Nebengruppe angewendet. Besondere Bedeutung bei den Elementen der achten Nebengruppe kommt den Elementen Palladium, Rhodium und Ruthenium zu, von Palladium sind die Komplexe mit Palladium-(0), Ruthenium-(O) und Rhodium-(I) die bevorzugten Oxidationsstufen, die sich vorzüglich für die C-O-C-Knüpfungsreaktion an den Hydroxylgruppen sowie für die Dimerisierung unter Wasserabspaltung eignen.

Besonders geeignet sind als Katalysatoren solche Komplexe, die hydrophile bzw. hydrophobe, chirale und achirale Phosphite, Phosphine und Diphosphine, aliphatische und aromatische Phosphine und Diphosphine alle Alkyl-, Alkenyl, Alkoxy, Aryl, substituierte Aryl-, Heteroaryl-, usw. Phosphine, Arylalky-phosphine der allgemeinen Formel: R¹R²R³P und Diphosphine der allgemeinen Formel: R¹R²P-(B)-PR³R⁴ enthalten.

Besonders geeignet sind Pd-haltige Katalysatoren, wobei das Element Palladium in verschiedenen Oxidationstufen als Pd(II)-, und Pd(0)-Verbindung wie Pd₂(OAc)₄, [Pd(COD)X]₂ (X = Halogen), [Pd(NBD)X]₂ (X = Halogen), Pd(COD)acac, Pd(Ph₃P)₄, Pd(dba)₂ als Vorstufe für die Katalysatoren verwendet wird.

Palladium-(0)-Komplex-Verbindungen können auf unterschiedlicher Weise hergestellt werden. Es ist möglich, sie aus wasserlöslichen Verbindungen wie PdCL₂ und Pd₂(OAc)₄ direkt oder aus anderen Komplexen wie [Pd(COD)Cl]₂, Pdacac₂, Pd(CH₃CN)₂Cl₂, Pd(C₆H₅CN)₂Cl₂ mit Liganden zu präparieren. Des Weiteren sind Palladium-(0)-Komplex-Verbindungen, die Imidazolinderivate enthalten, besonders gute Katalysatoren für Telomerisation hochaktiv. Diese Imidazolderivate sind äußerst effektiv, wenn sie als Carbenligand wirken.

Es gilt, dass solche Katalysatoren für die Telomerisation von Dienen an Hydroxyacetalen vor Vorteil sind die Pd(0)-Komplexen mit Imidazoolderivate als Liganden aufweisen, vorzugsweise mit einem Katalysatorkomplex [(Imes)Pd⁰(dvds)].

Ruthenium-(O)-Komplexe können aus Rutheniumverbindungen mit der Oxidationstufe 1 bis 3 hergestellt werden, wie z. B. Rutheniumhalogenverbindungen wasserfrei bzw. wasserhaltig, [Ru(CO)₂(CH₃COO)]ₓ, Ruacac₃, Ru₃(CO)₁₂, RuH(CO)₂(PPh₃)₃, RuCl(CO(PPh₃)₃ mit hochwirksamen Liganden für die Dimerisierungsreaktion. Der Einsatz von Phosphinen oder Phosphiten, die als Ligand genutzt und gleichzeitig die Reduktion zum Ruthenium-(0) ermöglichen. Die Reduktion kann auch von Lösungsmittel bzw. -systemen vorgenommen werden. Bevorzugte Ruthenverbindung sind RuX₃, RuX₃ x'y H₂O, (X = Halogen), (C₅H₅)₂Ru, (C₄H₇)₂(COD)Ru, (C₅H₅)RuCl(PPh₃)₂, (COD)RuCl₂, (C₁₂H₁₈)RuCl₂, (PPh₃)₃RuCl₂, Ru(acac)₃. K₂RuCl₅ x n H₂O, K₄Ru(CN)₆ X H₂O, Ru(NO)Cl₃ x n H₂O, (NO)Ru(NO₃)₃ und RuO₂ x n H₂O die durch Reaktion mit Phosphin- oder Phosphitverbindung herzustellen sind.

Rh(I)-Komplexverbindungen für die gewünschten Carbonylierungen lassen sich aus RhCl₃ x H₂O_{y}, Rhacac₂, Rhacac₃, RhOAc₂, RhOAc₃, Rh(COD)Cl, Rh(NBD)Cl, Rhₓ(CO)_{y}, Rh(COD)acac, Rh(NBD)acac, RhCl(CH₂=CH₂)₂ mit hochwirksamen Liganden für die Carbonylierung mit Phosphinen oder Phosphiten als Liganden herstellen. Jedoch sind auch die o. g. Palladium(0)-Komplexe für diese Reaktion einsetzbar.

Als Liganden in diesen Übergangsmetallkomplex-Katalysatoren können Phosphine und Phosphite, Diphoshpine bzw -phosphite, aber auch Imidazolderivate eingesetzt werden. Als Ligand Phosphin können alle kommerziell erhältlichen tertiären Phosphine und Phosphite, besonders (Ligand = Triaryl-, Diaryl-alkyl-, Aryl-dialkyl- oder Trialkylphosphin oder Diphosphine) Triaryl-, bedeutet im einfachsten Fall Triphenyl-, Diarylalkyl- Diphenylalkyl- bzw. Trialkyl- Tributyl-,Dibutylhexyl- aber auch Diadamantylbutylphosphin, genutzt werden. Besonders Triphenylphosphin sowie dessen Derivate und die stark basischen Phosphine eignen sich für die C-O-C-Knüpfungsreaktion. Wesentlich ist, dass das Übergangsmetall/Phosphor - Verhältnis in den beschriebenen Grenzen eingehalten wird.

### Hydroxyacetale

Das erfindungsgemäße Verfahren verwendet als Edukte die Hydroxyacetale der allgemeinen Formel (I), die vorzugsweise ausgewählt sind aus den Verbindungen 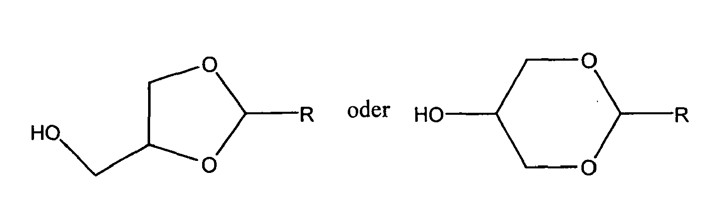 wobei R jeweils für einen linearen oder verzeigten Alkylrest mit 1 bis 12 C-Atomen steht und X eine optional vorhandene lineare oder verzweigt Alkylgruppe mit 1 bis 6 C-Atomen darstellt. Natürlich können auch Gemische der strukturverschiedenen Hydroxyacetale Verwendung finden.

Diese Verbindungen sind entweder kommerziell erhältlich, oder können in für den Fachmann bekannte Art und Weise synthetisiert werden.

Als C-O-C- bzw. C-O-C=O-Knüpfungsreaktionen ausgewählt sind vorzugsweise drei Reaktionstypen, nämlich (i) die Dimerisierung unter Wasserabspaltung, (ii) die Telomerisierung in Gegenwart von Olefinen und (iii) die Carbonylierung der Hydroxyacetale:

### (i) Dimerisierung unter Wasserabsalptung

Bei der Dimerisierung werden zwei Hydroxyacetale der allgemeinen Formel (I) in Gegenwart der Katalysatoren und unter Wasserabspaltung zur Reaktion gebracht, wie es beispielhaft im Schema 1 dargestellt ist:

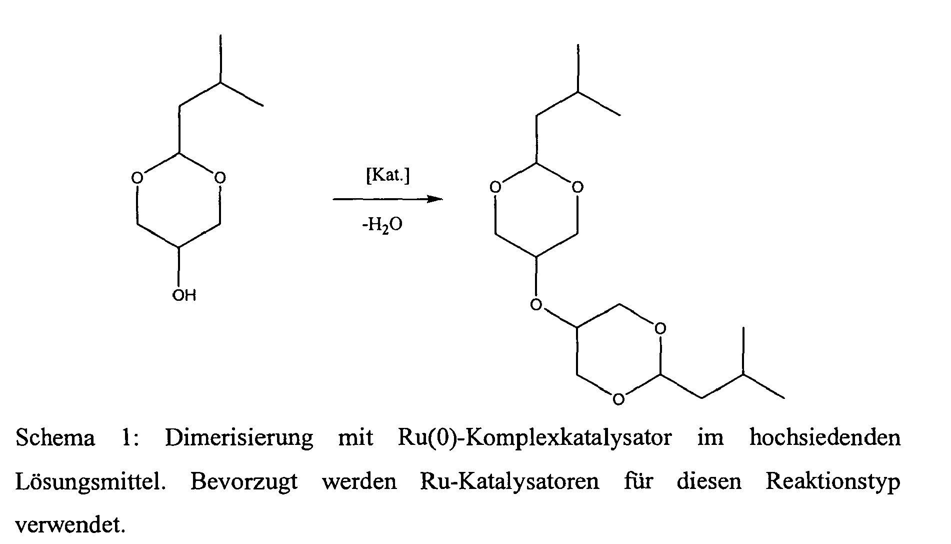

Die Dimerisierung der Hydroxyacetale unter Wasserabspaltung wird vorzugsweise in Gegenwart von Säuren, besonders von konzentrierten Säuren wie HCl, H₂SO₄ oder H₃PO₄ vorgenommen oder in Gegenwart von sauren Ionenaustauschern.

### (ii) Telomerisierung

Prinzipiell ist die Telomerisation von Dienen an Hydroxylgruppen bekannt [Tisch, M. EP 20070450078, Behr, A., "Angewandte homogene Katalyse", 2008, Wiley-VCH, Behr, A., et al., "Telomerisation - Fortschritte und Anwendungen einer vielseitigen Reaktion", 2009, 121, 3652-3669, P. J.C. Hausoul, P. C. A. Bruijnincx, R. J. M. Klein Gebbrink, B. M. Weckhuysen, Chem. Sus. Chem., 2009, 2, 855 - 858, u. a.]. Behr und Leschinski [Vortrag 7th Euro Fed Congress, Graz, 19.10.2009] nutzten die Telomerisation von Butadien von Glycerin, um Mono-, Di und Trioligomere herzustellen. In einem Mehrphasen Prozess haben sie gezeigt, dass mit Pd-TPPTS-Katalysatoren bis zu 90 % des Monotelomeren erhalten werden.

Jackstell et al. [R. Jackstell, M. G. Andreu, A. Frisch, K. Selvakumar, A. Zapf, H. Klein, A. Spannenberg, D. Roettger, O. Briel, R. Karch, M. Beller, Angew. Chem. 2002, 114, 6, 1028-1021, Angew. Chem. Int. Ed. 2002, 41, 6, 986-989,] verwenden Imidazolderivate für eine äußerst effektive Telomerisation an einfachen Alkoholen.

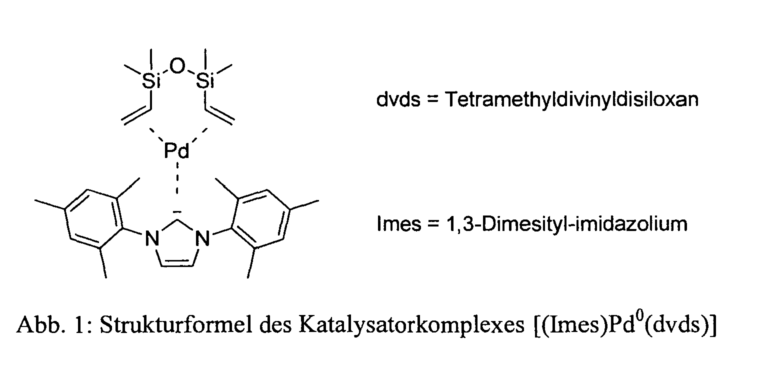

Als Katalysator wird bevorzugt ein Palladium(0)carbenkomplex (Abb. 1) verwendet. Überraschender Weise lassen sich dieser in Abb. 1 gezeigten Palladium(0)-carbenkomplex für die Telomerisation von 1,3 Butadien bzw. Isopren auch auf Hydroxylgruppen haltige Glycerinacetale und -gemische anwenden. Dieser Komplex zeigt im Vergleich zu *in-situ* Systemen aus Pd(acac)₂ und Liganden, die durch Deprotonierung zu Carbenen umgesetzt werden, eine höhere Aktivität. Sehr geringe Katalysatormengen (9,4 x 10⁻⁶mol) werden eingesetzt. Bei einem Verhältnis von 20.000 Butadien/Palladium werden ungefähr 80 % der gewünschten Telomerisationsprodukte erhalten. TON's (= turnover number) von 20.000 und mehr werden erreicht. Die TON gibt die dabei Anzahl von Molen Substrat an, die bis zur Erschöpfung eines Mols an Katalysator umgesetzt werden können.

Das nachfolgende Reaktionsschema verdeutlicht die Abfolge der Reaktion:

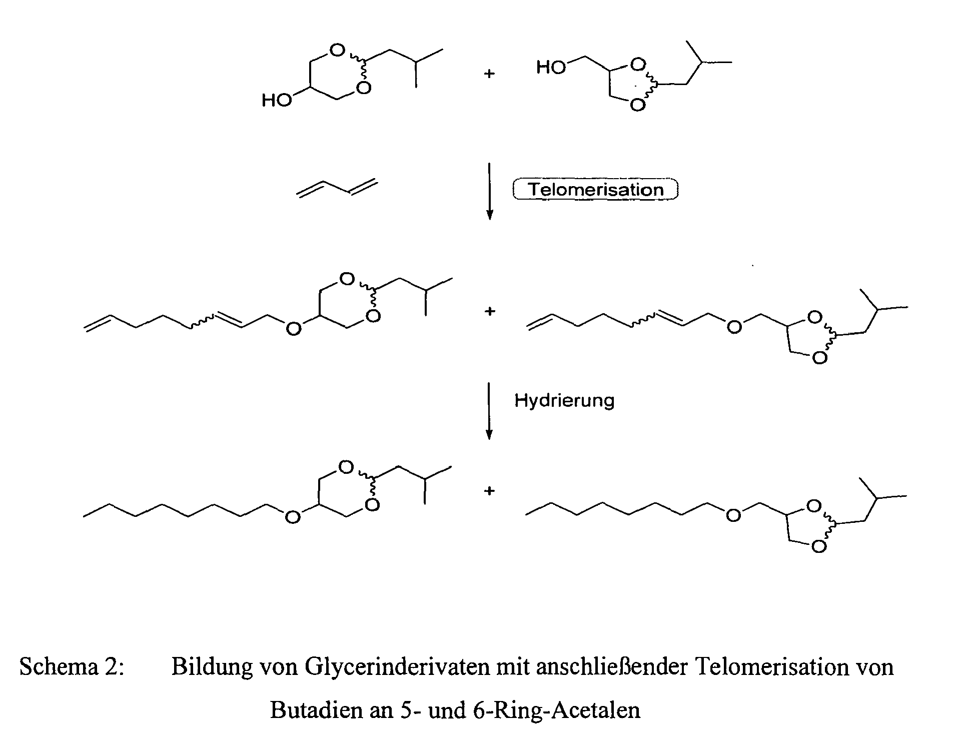

Das Acetal bzw, das Acetalgemisch wird vorzugsweise in einem hoch siedenden Lösungsmittel, wie Cyclohexan, Tetrachlorkohlenstoff, Trichlormethan, Toluol, Xylol, Diphenylether oder Gemischen oder ohne Lösungsmittel aus selbigem, mit einem Ruthenium(O)-Phosphinkomplex, gebildet aus RuCl₃, und einem basischem Liganden von Phosphin bzw. Phosphit, mit wasserentziehenden Substanzen wie Schwefelsäure, Phosphorsäure oder sauren Molsiebe unter Rückfluss in Argonatmosphäre gekocht. Das Hydroxyacetal dimerisiert unter Wasserabspaltung. Als Ligand Phosphin können alle kommerziell erhältlichen tertiären Phosphine und Phosphite, besonders (Ligand = Triaryl-, Diaryl-alkyl-, Aryl-dialkyl- oder Trialkylphosphin oder Diphosphine) Triaryl-, bedeutet im einfachsten Fall Triphenyl-, Diarylalkyl- Diphenylalkyl- bzw. Trialkyl- Tri-n-butyl-, Tri-t-butyl, Dibutylhexylaber auch Diadamantylbutylphosphin, genutzt werden. Im Anschluss an die Telomerisierung erfolgt vorzugsweise noch eine Hydrierungsreaktion des Zwischenprodukts, wobei vorzugsweise der gleiche Katalysator wie bei der Telomerisierung zum Einsatz kommt oder das eine getrennte Katalyse mit heterogenen Katalysatoren vorgenommen wird, die strukturell anders sind als die zur Telomerisierung eingesetzten Verbindungen.

Es kann von Vorteil sein, dass die Katalysatoren für die Telomerisation von Dienen an Hydroxyacetalen neben den Imidazolderivate auch Siloxane und vorzugsweise Tetrametyldivinylsiloxane enthalten.

### (iii) Carbonylierung

Die Carbonylierung von Aryl-, Vinyl- und Benzylhalogenen ist unter CO mit PdCl₂-Komplexen bekannt [B. Cornils, A. Herrmann, Applied Homogeneous Catalysis with Organometallic Compounds, VCH , Weinheim, 1996, 187, M. Beller, J. G. E. Krauter, in Multiphase Homogeneous Catalysis, B. Cornils, A. Herrmann, I. T. Horvath, W. Leitner, S. Mecking, H. Olivier-Bourbigou, D. Vogt (Eds.), Wiley-VCH, 2005, 183].

Die Carbonylierung wird vorzugsweise im Autoklav in einem polaren Lösungsmittel bei Temperaturen zwischen 50 und 150 °C in Gegenwart von Pd(0)-Komplexen vorgenommen. Werden Halogenverbindungen der Carbonylierung unterworfen ist die Nutzung von Basen, bevorzugt in einer zweiten Phase, notwendig. Es besteht die Möglichkeit, analog zur Hydroformylierung, Olefine ein- und umzusetzen. In diesem Fall werden Aldehyde synthetisiert.

Erfindungsgemäß werden aus den Olefinen durch Carbonylierung Aldehyde gebildet, die in einer Folgereaktion mit Hydroxylgruppen haltigen Monocyloacetalen bzw. - gemischen zu Carbonsäureestern abreagieren. Die Carbonylierung wird vorzugsweise in Autoklaven bei Drucken zwischen 20 und 100 bar CO durchgeführt.

Das folgende Schema verdeutlicht den Reaktionsablauf:

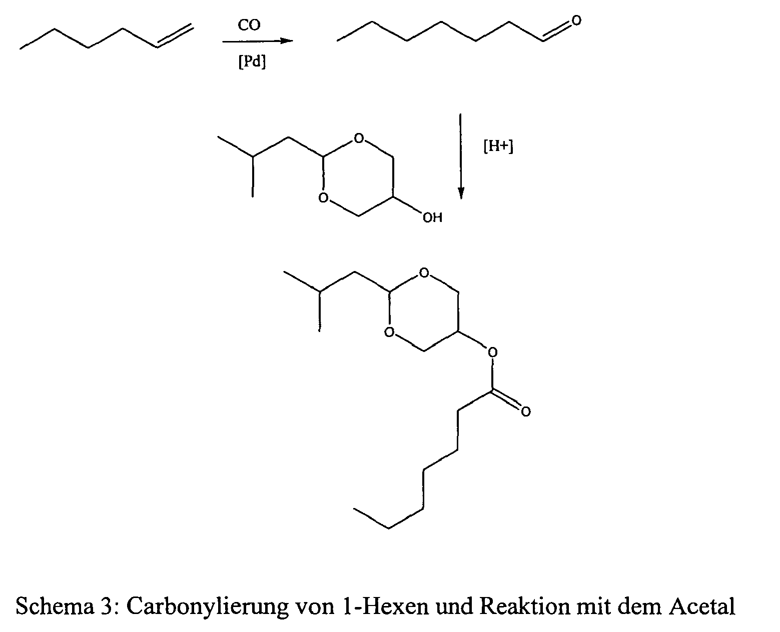

Für die Carbonylierung werden vorzugsweise Olefine mit C₂- bis C₁₈-Atomen, und vorzugsweise als reine 1-Olefine, 2-Olefine oder auch als technische Olefingemische eingesetzt und die Umsetzungen erfolgt vorzugsweise unter einem Kohlenmonoxid Gesamtdruck zwischen 1 und 300 bar, bevorzugt zwischen 1 und 100 bar.

Die Olefine können auch weitere Heterogruppen wie beispielsweise sauerstoff-, stickstoff- oder/und schwefelhaltige Substituenten enthalten.

Allgemein gilt, dass soweit bei den oben beschriebenen Synthesen Mehrphasensysteme zum Einsatz kommen, polare/apolare Mehrphasenlösungsmittelsysteme, besonders ohne Lösungsmittel oder mit DMF, THF, Diethylether, Dimethylether, DMSO, Methanol, Ethanol, Propanol, Hexan, Heptan, Octan, Decan, Benzen, Toluen, Xylen, Petrolbenzin und -gemische, Tetrachlorkohlenstoff, Chloroform, Dichlormethan, Trichlorethylen, Dichlorethan, Aceton, Acetonitril und Essigsäureethylester nebst Polyolen und Wasser, bevorzugt zur Anwendung kommen.

Die Reaktionstemperaturen liegen vorzugsweise zwischen 0 und 200 °C, und bevorzugt zwischen 25 und 150 °C.

Ein weiterer Aspekt der vorliegenden Erfindung betrifft die Verwendung der in einem Verfahren nach der obigen Beschreibung hergestellten Produkten als Additiv in Kraftstoffen, insbesondere in Benzin- bzw. Dieselkraftstoffen oder deren Gemischen, als Lösungsmittel, Detergenzien, Kosmetika, Weichmacher, Stabilisatoren, pharmazeutische Agenzien, Fungizide und Mikrobiozide oder Aromastoffe, wobei die Verwendung als Additiv in Krafftstoffen wie Dieselöl oder Benzin besonders bevorzugt sein kann.

### Beispiele

### Beispiel 1: Telomerisation von Diolefinen am Hydroxyacetal

5.6 mg (9,38 10⁻⁶ mol) [(Imes)Pd(dvds)] werden in 50 mL THF unter Argon gelöst. Es werden 0,5 mol-% KOH und 30 mL der Acetalmischung zugesetzt. Die Lösung wird unter Argon in einem 300 mL-Autoklaven der Firma *Parr* überführt. Der Autoklav wird mit Ethanol und Trockeneis gekühlt. 10 g (0,188 mol) 1,3-Butadien werden in einen separaten 75-mL-Druckzylinder kondensiert. Das 1,3-Butadien wird in den gekühlten Autoklaven überführt, bei 90°C wird telomerisiert. Auf Raumtemperatur gekühlt, nicht umgesetztes 1,3-Butadien entfernt. 2,2,4-Trimethylpentan dient als interner Standard. Die Ausbeute an Telomerisationsprodukten wird gaschromatographisch bestimmt (R_{f} = 1,1737). Das Produktgemisch wird im Vakuum destilliert (Kp: _{0,01 mbar} = 95-100 °C).

### Beispiel 2: Telomerisation von Diolefinen am Hydroxyacetal

5.6 mg (9,38 10⁻⁶ mol) [(Imes)Pd(dvds)] und Ligand werden in 50 mL THF unter Argon gelöst. Es werden 0,5 mol-% KOH und 30 mL der Acetalmischung zugesetzt. Die Lösung wird unter Argon in einem 300 mL-Autoklaven der Firma *Parr* überführt. Der Autoklav wird mit Ethanol und Trockeneis gekühlt. 10 oder 20 g (0,188 oder 0,375 mol) 1,3-Butadien werden in einen separaten 75-mL-Druckzylinder kondensiert. Das 1,3-Butadien wird in den gekühlten Autoklaven überführt, bei 90 °C wird telomerisiert. Auf Raumtemperatur gekühlt, nicht umgesetztes 1,3-Butadien entfernt. 2,2,4-Trimethylpentan dient als interner Standard. Die Ausbeute an Telomerisationsprodukten wird gaschromatographisch bestimmt (R_{f} = 1,1737). Das Produktgemisch wird im Vakuum destilliert (Kp: _{0,01 mbar} = 95-100 °C).

### Beispiel 3: Telomerisation von Diolefinen am Hydroxyacetal

5.6 mg (9,38 10⁻⁶ mol) [(Imes)Pd(dvds)] und Ligand, 20 Äq. ImesMeSO₃ werden in 50 mL THF unter Argon gelöst. Es werden 0,5 mol-% KOH und 30 mL der Acetalmischung zugesetzt. Die Lösung wird unter Argon in einem 300 mL-Autoklaven der Firma *Parr* überführt. Der Autoklav wird mit Ethanol und Trockeneis gekühlt. 20 g (0,375 mol) 1,3-Butadien werden in einen separaten 75-mL-Druckzylinder kondensiert. Das 1,3-Butadien wird in den gekühlten Autoklaven überführt, bei 90°C wird telomerisiert. Auf Raumtemperatur gekühlt, nicht umgesetztes 1,3-Butadien entfernt. 2,2,4-Trimethylpentan dient als interner Standard. Die Ausbeute an Telomerisationsprodukten wird gaschromatographisch bestimmt (R_{f} = 1,1737). Das Produktgemisch wird im Vakuum destilliert (Kp: _{0,01 mbar} = 95-100 °C).

### Beispiel 4: Telomerisation von Diolefinen am Hydroxyacetal

2,85 mg (9,38*10⁻⁶ mol) Pd(acac)₂ und 10 Äq. ImesC1 werden in 50 mL THF unter Argon gelöst. Es werden 0,5 mol-% KOH und 30 mL der Acetalmischung zugesetzt. Die Lösung wird unter Argon in einem 300 mL-Autoklaven der Firma *Parr* überführt. Der Autoklav wird mit Ethanol und Trockeneis gekühlt. 20 g (0,375 mol) 1,3-Butadien werden in einen separaten 75-mL-Druckzylinder kondensiert. Das 1,3-Butadien wird in den gekühlten Autoklaven überführt, bei 90 °C wird telomerisiert. Auf Raumtemperatur gekühlt, nicht umgesetztes 1,3-Butadien entfernt. 2,2,4-Trimethylpentan dient als interner Standard. Die Ausbeute an Telomerisationsprodukten wird gaschromatographisch bestimmt (R_{f}= 1,1737). Das Produktgemisch wird im Vakuum destilliert (Kp: _{0,01 mbar} = 95-100 °C).

### Beispiel 5: Telomerisation von Diolefinen am Hydroxyacetal

2,85 mg (9,38* 10⁻⁶ mol) Pd(acac)₂ und 10 Äq. ImesMeSO₃ werden in 50 mL THF unter Argon gelöst. Es werden 0,5 mol-% KOH und 30 mL der Acetalmischung zugesetzt. Die Lösung wird unter Argon in einem 300 mL-Autoklaven der Firma *Parr* überführt. Der Autoklav wird mit Ethanol und Trockeneis gekühlt. 20 g (0,375 mol) 1,3-Butadien werden in einen separaten 75-mL-Druckzylinder kondensiert. Das 1,3-Butadien wird in den gekühlten Autoklaven überführt, bei 90 °C wird telomerisiert. Auf Raumtemperatur gekühlt, nicht umgesetztes 1,3-Butadien entfernt. 2,2,4-Trimethylpentan dient als interner Standard. Die Ausbeute an Telomerisationsprodukten wird gaschromatographisch bestimmt (R_{f}= 1,1737). Das Produktgemisch wird im Vakuum destilliert (Kp: _{0,01 mbar} = 95-100 °C).

### Beispiel 6: Carbonylierung von Olefinen

In einen 100-ml-Autoklaven wurden 20 ml Dioxan, 0,05 mol (4,2 g) 1-Hexen und 0,05 mol (8 g) Acetal gegeben. 50 mg Pd(OAc)₂ und 500 mg Di-Adamantylphosphin wurden für die Carbonylierung des Hexens, sowie 0,10 g p-Toluolsulfonsäure für die anschließende Veresterung beigefügt. Das Reaktionsgemisch wurde für 24 h bei 100 °C und 50 bar CO-Druck gerührt.

Das Dioxan, sowie Reste des Hexens am Rotationsverdampfer abgezogen. Das Produkt über Kieselgel gereinigt.

### Beispiel 7: Carbonylierung von Olefinen

In einen 100-ml-Autoklaven wurden 20 ml Dioxan, 0,05 mol (7,87 ml) 1-Octen und 0,05 mol (8 g) Acetal gegeben. 50 mg Pd(OAc)₂ und 500 mg Di-adamantyl-n-butylphosphin wurden für die Carbonylierung des Hexens, sowie 0,10 g p-Toluolsulfonsäure für die anschließende Veresterung beigefügt. Das Reaktionsgemisch wurde für 24 h bei 100 °C und 50 bar CO-Druck gerührt.

Das Dioxan, sowie Reste des Hexens am Rotationsverdampfer abgezogen. Das Produkt über Kieselgel gereinigt.

### Beispiel 8: Carbonylierung von Olefinen

In einen 100-ml-Autoklaven wurden 20 ml Dioxan, 0,05 mol (7,87 ml) 1-Octen und 0,05 mol (8 g) Acetal gegeben. 50 mg Pd(OAc)₂ und 500 mg Di-adamantyl-n-butylphosphin wurden für die Carbonylierung des Hexens, sowie 0,10 g p-Toluolsulfonsäure für die anschließende Veresterung beigefügt. Das Reaktionsgemisch wurde für 24 h bei 120 °C und 20 bar CO-Druck gerührt.

Das Dioxan, sowie Reste des Hexens am Rotationsverdampfer abgezogen. Das Produkt über Kieselgel gereinigt.

### Beispiel 9 ; Dimerisierung von Hydroxyacetalgemischen

In einen 100-ml-Kolben wurden 5 g Acetalgemisch, 20 ml Xylol, 65 mg RuCl₃, 500 mg Di-Adamantylphosphin und einige Molsiebe gegeben. Das Gemisch wurde über Stunden unter Rückfluss und Argonatmosphäre gekocht. Nach 48 h wurde mittels GC eine Ausbeute von Dimeren von 30 % gefunden.

### Beispiel 10 ; Dimerisierung von Hydroxyacetalgemischen

In einen 100-ml-Kolben wurden 5 g Acetalgemisch, 65 mg RuCl₃, 500 mg Di-Adamantylphosphin und einige Molsiebe gegeben. Das Gemisch wurde über Stunden auf 160 °C und Argonatmosphäre erwärmt. Nach 48 h wurde mittels GC eine Ausbeute von Dimeren von 57 % gefunden.

## Patentansprüche

1. Verfahren zur chemischen Reaktion von Hydroxyacetalen der allgemeinen
Formel (I) 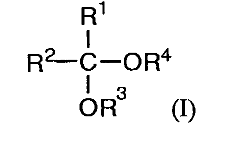 wobei die Gruppen R¹, R², R³ und R⁴ für lineare, verzweigte, cyclische und ggf. untereinander verknüpfte Alkylgruppen steht, mit der Maßgabe, dass mindestens eine der Gruppen R¹, R², R³ oder R⁴ eine freie Hydroxylgruppe aufweist, bzw. Gemische von Verbindungen der allgemeinen Formel (I) durch C-O-C- bzw. C-O-C=O-Knüpfungsreaktionen der Hydroxylfunktion der Verbindungen der allgemeinen Formel (I) oder deren Gemische in Gegenwart von Übergangsmetall-Phosphin- bzw. -Phosphit-Komplexkatalysatoren der 8. Nebengruppe in Ein- oder Mehrphasensystemen, **dadurch gekennzeichnet, dass** die Knüpfungsreaktion bei Temperaturen zwischen 0 und 200 °C durchgeführt wird, wobei für die Komplexkatalysatoren die Übergangsmetall/Phosphor-Verhältnisse zwischen 1 : 0,5 und 1 : 100 betragen.

2. Verfahren nach Anspruch 1, **dadurch gekennzeichnet, dass** die Hydroxyacetale
der allgemeinen Formel (I) ausgewählt sind aus den Verbindungen 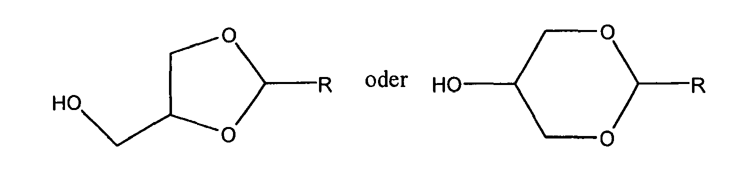 wobei R jeweils für einen linearen oder verzeigten Alkylrest mit 1 bis 12 C-Atomen steht und X eine optional vorhandene lineare oder verzweigt Alkylgruppe mit 1 bis 6 C-Atomen darstellt.

3. Verfahren nach den Ansprüchen 1 bis 2, **dadurch gekennzeichnet, dass** als C-O-C- bzw. C-O-C=O-Knüpfungsreaktionen ausgewählt sind (i) Dimerisierung unter Wasserabspaltung, (ii) Telomerisierung , (iii) Carbonylierung.

4. Verfahren nach den Ansprüchen 1 bis 3, **dadurch gekennzeichnet, dass** als Übergangsmetalle Pd, Ru und Rd, vorzugsweise in der Oxidationsstufe 0 oder +1 ausgewählt sind.

5. Verfahren nach den Ansprüchen 1 bis 4, **dadurch gekennzeichnet, dass** als Katalysatoren hydrophile bzw. hydrophobe, chirale und achirale Phosphite, Phosphine und Diphosphine, aliphatische und aromatische Phosphine und Diphosphine alle Alkyl-, Alkenyl, Alkoxy, Aryl, substituierte Aryl-, Heteroaryl-, usw. Phosphine, Arylalky-phosphine der allgemeinen Formel: R¹R²R³P und Diphosphine der allgemeinen Formel: R¹R²P-(B)-PR³R⁴ sein können.

6. Verfahren nach Anspruch 1 bis 5, **dadurch gekennzeichnet, dass** das Element Palladium in verschiedenen Oxidationstufen als Pd(II)-, und Pd(0)-Verbindung wie Pd₂(OAc)₄, [Pd(COD)X]₂ (X = Halogen), [Pd(NBD)X]₂ (X = Halogen), Pd(COD)acac, Pd(Ph₃P)₄, Pd(dba)₂ als Vorstufe für die Katalysatoren verwendet wird.

7. Verfahren nach den Ansprüchen 1 bis 6, **dadurch gekennzeichnet, dass** für die Telomerisation von Dienen an Hydroxyacetalen Pd(0)-Komplexen mit Imidazolderivate als Liganden erfolgt, vorzugsweise mit einem Katalysatorkomplex [(Imes)Pd⁰(dvds)].

8. Verfahren nach Anspruch 1 bis 5, **dadurch gekennzeichnet, dass** die Ruthenium(0)komplexe aus verschiedenen Ruthenverbindung RuX₃, RuX₃ x'y H₂O, (X = Halogen), (C₅H₅)₂Ru, (C₄H₇)₂(COD)Ru, (C₅H₅)RuCl(PPh₃)₂, (COD)RuCl₂, (C₁₂H₁₈)RuCl₂, (PPh₃)₃RuCl₂, Ru(acac)₃. K₂RuCl₅ x n H₂O, K₄Ru(CN)₆ X H₂O, Ru(NO)Cl₃ x n H₂O, (NO)Ru(NO₃)₃ und RuO₂ x n H₂O durch Reaktion mit Phosphin- oder Phosphitverbindung herzustellen sind.

9. Verfahren nach Anspruch 7, **dadurch gekennzeichnet, dass** die Katalysatoren für die Telomerisation von Dienen an Hydroxyacetalen neben den Imidazolderivate auch Siloxane und vorzugsweise Tetrametyldivinylsiloxane enthalten.

10. Verfahren nach Anspruch 1 bis 9, **dadurch gekennzeichnet, dass** sich nach der Telomerisation eine Hydrierung der Produkte vorzugsweise mit den gleichen Pd-Katalysatoren anschließen kann.

11. Verfahren nach Anspruch 1 bis 10, **dadurch gekennzeichnet, dass** die Hydrierung nach der Telomerisation in einer getrennten Katalyse mit heterogenen Katalysatoren vorgenommen werden kann.

12. Verfahren nach Anspruch 1 bis 11, **dadurch gekennzeichnet, dass** für Carbonylierung Olefine mit C₂- bis C₁₈-Atomen, und vorzugsweise als reine 1-Olefine, 2-Olefine oder auch als technische Olefingemische eingesetzt werden und die Umsetzungen unter einem Kohlenmonoxid Gesamtdruck zwischen 1 und 300 bar, bevorzugt zwischen 1 und 100 bar, erfolgt.

13. Verfahren nach Anspruch 12, **dadurch gekennzeichnet, dass** als 1-Olefine , innere Olefine, innere als cis- oder trans-Isomere Olefine, oder technische Olefingemische ausgewählt werden.

14. Verfahren nach Anspruch 12 bis 13, **dadurch gekennzeichnet, dass** die Olefine auch weitere Heterogruppen wie beispielsweise sauerstoff-, stickstoff- oder/und schwefelhaltige Substituenten enthalten können.

15. Verfahren nach Anspruch 1 bis 14, **dadurch gekennzeichnet, dass** die Dimerisierung von Hydroxyacetalen bzw. -gemischen mit Rutheniumkomplexkatalysatoren unter Wasserabspaltung bzw. -entfernung vorgenommen werden.

16. Verfahren nach Anspruch 1 bis 15, **dadurch gekennzeichnet, dass** Mehrphasensysteme polare/apolare Mehrphasenlösungsmittelsysteme,
besonders ohne Lösungsmittel oder mit DMF, THF, Diethylether, Dimethylether, DMSO, Methanol, Ethanol, Propanol, Hexan, Heptan, Octan, Decan, Benzen, Toluen, Xylen, Petrolbenzin und -gemische, Tetrachlorkohlenstoff, Chloroform, Dichlormethan, Trichlorethylen, Dichlorethan, Aceton, Acetonitril und Essigsäureethylester nebst Polyolen und Wasser, in Anwendung kommen.

17. Verfahren nach Anspruch 1 bis 16, **dadurch gekennzeichnet, dass** Ein- bzw.
Mehrphasensysteme als polare/apolare Mehrphasensysteme ohne weitere Lösungsmittel eingesetzt werden können.

18. Verfahren nach Anspruch 1 bis 17, **dadurch gekennzeichnet, dass** die Reaktionstemperatur zwischen 0 und 200 °C, und bevorzugt zwischen 25 und 150 °C liegt.

19. Verfahren nach den Ansprüchen 1 bis 18, **dadurch gekennzeichnet, dass** die Dimerisierung der Hydroxyacetale unter Wasserabspaltung in Gegenwart von Säuren, besonders von konzentrierten Säuren wie HCl, H₂SO₄ oder H₃PO₄ vorgenommen wird.

20. Verfahren nach den Ansprüchen 1 bis 19, **dadurch gekennzeichnet, dass** die Dimerisierung der Hydroxyacetale unter Wasserabspaltung in Gegenwart von sauren Ionenaustauschern vorgenommen wird.

21. Verwendung der in einem Verfahren nach den Ansprüchen 1 bis 20 hergestellten Produkten als Additiv in Benzin- bzw. Dieselkraftstoffgemischen, als Lösungsmittel, Detergenzien, Kosmetika, Weichmacher, Stabilisatoren, pharmazeutische Agenzien, Fungizide und Mikrobiozide oder Aromastoffe.
